# EUROPÄISCHE PATENTSCHRIFT

(11) **EP 1 341 459 B1**
(45) Veröffentlichungstag und Bekanntmachung des Hinweises auf die Patenterteilung: **10.08.2005**
(21) Anmeldenummer: 01991801.0
(22) Anmeldetag: 07.12.2001
(51) Int. Cl.: A61B 18/08, A61B 18/14, H01H 1/00, A61B 18/10, A61B 17/30, C02F 1/461

(54) **INSTRUMENT FÜR CHIRURGISCHE ZWECKE MIT KONTAKTBEREICHEN AUS DOTIERTEM DIAMANT SOWIE VERFAHREN ZU DESSEN REINIGUNG**
INSTRUMENT FOR SURGICAL APPLICATIONS COMPRISING DOPED DIAMOND CONTACT AREAS AND METHOD FOR CLEANING THE SAME
INSTRUMENT POUR APPLICATIONS CHIRURGICALES MUNIE D'UNE ZONE DE CONTACT AU DIAMANT DOP ZONES AVEC DIAMANT DOP ET PROCEDE DE NETTOYAGE DUDIT INSTRUMENT

(30) Priorität: 08.12.2000 DE 10061278
(43) Veröffentlichungstag der Anmeldung: 10.09.2003
(73) Patentinhaber: GFD Gesellschaft für Diamantprodukte mbH, 89081 Ulm (DE); Fraunhofer-Gesellschaft zur Förderung der angewandten Forschung e.V., 80686 München (DE)
(72) Erfinder: LINGENFELDER, Christian, 89081 Ulm (DE); ERTL, Stephan, 89075 Ulm (DE); STROBEL, Stefan, 89129 Langenau (DE); RÖSCH, Rudolf, 89335 Ichenhausen (DE); FÖRSTER, Siegfried, 89231 Neu-Ulm (DE); FRYDA, Matthias, 25524 Itzehoe (DE); SCHÄFER, Lothar, 38527 Meine (DE); TRÖSTER, Inga, 38118 Braunschweig (DE); HERRMANN, Dennie, 38116 Braunschweig (DE)
(74) Vertreter: Pfenning, Meinig & Partner
(86) Internationale Anmeldenummer: PCT/EP2001/014373
(87) Internationale Veröffentlichungsnummer: WO 2002/045589

(56) Entgegenhaltungen:
- EP-A- 0 994 074
- WO-A-00/44012
- WO-A-01/50964
- WO-A-01/89402
- WO-A-99/40858
- DE-A- 19 652 098
- US-A- 6 066 137

## Beschreibung

Die vorliegende Erfindung bezieht sich auf ein Instrument für chirurgische Zwecke, insbesondere zur Koagulation, nach dem Oberbegriff des Anspruches 1 sowie ein Verfahren zu dessen Reinigung.

In der Chirurgie bzw. in der Mikrochirurgie hat die Koagulation ein sehr weites Indikationsgebiet. Herkömmlicherweise wird die Koagulation zum Stoppen von Blutungen an offenen Wunden mit Hilfe einer Zange, einer Pinzette, einem Draht oder einer Klinge bewirkt. Beispielsweise wird das blutende Gefäß mit der Zange oder der Pinzette zusammengedrückt und dann ein Hochfrequenzstrom durch die Spitze geführt (bipolare Anwendung).

Der Hochfrequenzstrom kann auch z.B. über eine Klinge in das umgebende Gewebe eingeleitet werden. Als Gegenelektrode fungiert in diesem Falle eine großflächige Elektrode; der Stromfluß erfolgt durch den Körper, die Koagulation erfolgt im Klingenbereich, d.h. dem Bereich höchster Stromdichte (monopolare Anwendung).

In beiden Fällen verursacht der elektrische Widerstand des Gewebes eine Umsetzung der elektrischen Energie in thermische Energie und damit die Aufheizung und Koagulation des Gewebes, d.h. die Gerinnung der Zellsubstanz.

Es sind z.B. Pinzetten oder Zangen für chirurgische Zwecke bekannt, welche zur mono- und bipolaren Koagulation geeignet sind und zwei zueinander bewegbare Schenkel aufweisen, die elektrisch gegeneinander isoliert sind. Diese Schenkel weisen an ihren Spitzen elektrische Kontaktflächen aus Metall auf. Diese elektrischen Kontakte sind mit einem Steuergerät verbunden. Bei Berührung/Erfassen eines Stücks Gewebe zwischen den einander zugewandten Innenseiten und Aktivierung eines Stromflusses durch die Kontakte bzw. das Gewebe hindurch, kommt es zur Koagulation des erfaßten Gewebes.

Diese chirurgischen Instrumente nach dem Stand der Technik weisen mehrere Nachteile auf.

Oftmals kommt es bei der Verwendung von Metallen zu einem ungewünschten Anhaften des Gewebes an der Metalloberfläche, welches im schlimmsten Falle das Wiederaufreißen der koagulierten Stelle bewirken kann.

Außerdem ist die Verwendung herkömmlicher chirurgischer Instrumente mit Kontaktflächen aus Metall kostenaufwendig, da entweder bereits nach einer Operation die Instrumente verworfen werden oder aufwendig mechanisch und chemisch gereinigt werden müssen, wobei hierbei immer noch die Gefahr besteht, daß z.B. Gewebe- oder Blutreste zurückbleiben.

Hierbei geht von den verschmutzten Elektroden eine besonders hohe Gefahr aus. Angetrocknete Blut- oder Gewebereste genügen hierbei zur elektrischen Isolation der Metallkontaktflächen, es kann zunächst zur Stromunterbrechung und dann zu einer Funkenbildung bzw. Verkohlung der Kontaktfläche und Verbrennungen von Gewebe auf der Kontaktfläche kommen. Außerdem entstehen Zusatzgefahren bei Anhaftung infektiösen Gewebes.

Außerdem kann die begrenzte Wärmeleitfähigkeit des verwendeten Metalles dazu führen, daß es zu einer ungleichmäßigen Erwärmung des Metalles während des Koagulationsvorganges kommt und sich örtliche Temperaturspitzen ("Hot Spots") ausbilden. Es ist zu vermuten, daß an diesen "Hot Spots" schon bei normalem Gebrauch verbranntes Gewebe entsteht und haften bleibt. Dies bedeutet eine gefährliche Qualitätseinbuße bei Pinzetten nach dem Stand der Technik.

Auch die Wärmekapazität von Metallen kann sich nachteilig auswirken, da durch die im Metall gespeicherte Restwärme der Koagulationsvorgang unter Umständen länger als vom Operateur gewünscht fortgeführt wird, so daß es zu einer ungewünschten "Trägheit" des Gerätes kommt, welche Verbrennungen bzw. Anhaftungen verursachen kann, was insbesondere in der Mikrochirurgie sehr nachteilhaft ist.

Die gattungsgemäße WO 99/40858 beschreibt ein Instrument für chirurgische Zwecke mit einem Schenkel, der mit einer Stromquelle elektrisch verbindbar ist und zum Schneiden von biologischem Gewebe verwendet wird. Der Schenkel ist mit einem diamantähnlichen, elektrisch leitfähigem Material beschichtet.

Die zum allgemeinen Stand der Technik gehörende PCT/WO 00/44012 betrifft einen mechanisch schließenden elektrischen Mikroschalterkontakt mit zwei elektrisch leitenden Kontaktelementen, die zur Kontaktierung mit jeweiligen Kontaktflächen miteinander in Berührung treten. Erfindungsgemäß besteht nun mindestens eine der Kontaktflächen zumindest teilweise aus hochdotiertem leitfähigen Diamant, Siliciumcarbid, Galliumnitrit, Bornitrit, Aluminium-Galliumnitrit und/oder Aluminiumnitrit.

Der vorliegenden Erfindung liegt daher die Aufgabe zugrunde, ein Instrument zu schaffen, welches die oben bezeichneten Nachteile herkömmlicher Instrumente, insbesondere in Bezug auf die Entstehung und Beseitigung von Anhaftungen überwindet.

Diese Aufgabe wird bezüglich des Instruments durch Anspruch 1, bezüglich des Verfahrens durch Anspruch 26 gelöst.

Dadurch, daß die Kontaktbereiche aus einem zur Herstellung der elektrischen Leitfähigkeit dotierten Diamant bestehen, werden die Nachteile herkömmlicher Kontakte in Bezug auf Wärmeleitfähigkeit, Wärmekapazität, fehlende Inerteigenschaften bzw. ungewünschtes Anhaften sowie Verschmutzungen auf den Kontaktbereichen und schlechte Reinigbarkeit und einem daraus resultierendem ungleichmäßigen Stromfluß vermieden. Die Koagulation kann hierbei entweder durch direkten Kontakt des Gewebes mit der Diamantelektrode und dem dabei bestehenden Stromfluß durch das Gewebe hindurch oder durch kapazitive Energieübertragung, wie bei einer elektrisch beaufschlagten isolierten Elektrode, geschehen. In jedem Falle wird vor allem die Reinigbarkeit der Kontaktbereiche bzw. des Instruments stark verbessert und die Gefahr des Anhaftens von Gewebe verringert.

Diamant hat den Vorteil, daß er chemisch vollkommen inert und damit biokompatibel ist. Aufgrund des physikalischen Aufbaus kann es zu keiner Diffusion von Dotierstoffen aus dem Diamant heraus kommen. Somit ist der Einsatz in der Chirurgie bedenkenlos. Neben den Inerteigenschaften von Diamant an der Oberfläche des Kontaktbereiches ermöglicht die Terminierbarkeit (d.h. gezieltes Aufbringen chemisorbierter Moleküle oder Atome - wie beispielsweise Sauerstoff, Fluor (hydrophil) oder Wasserstoff (hydrophob) - auf die Oberfläche) der Diamant-Oberfläche die gezielte Einstellung der physikalischen und chemischen Eigenschaften der Oberfläche wie beispielsweise deren Hydrophobizität/Hydrophilität. Durch geeignete Terminierung der Oberfläche kann die Anhaftung von Gewebe weiter reduziert oder vermieden werden.

Auch die Wärmeleitfähigkeitseigenschaften werden drastisch verbessert. Dies liegt daran, daß Diamant als Isolator und dotiert als Leiter eine außerordentlich hohe Wärmeleitfähigkeit aufweist, welche sogar noch deutlich höher ist als die von Metallen wie Kupfer oder Silber. Hierdurch kommt es schnell zu einer gleichmäßigen Wärmeverteilung innerhalb des Kontaktbereiches, es treten keine hohen Temperaturgradienten auf, welche eine Hot-Spot-Bildung verursachen könnten.

Auch die Wärmekapazität des diamantenen Kontaktbereiches kann sehr gering gehalten werden. Diamant weist zunächst einmal eine geringe spezifische Wärmekapazität auf, welche praktisch unabhängig von der Dotierung ist. Außerdem kann Diamant in dünnen Schichten und trotzdem stabil ageschieden werden, so daß sich eine sehr geringe thermisch aktive Masse ergibt.

Die besondere Eignung von Kontaktbereichen aus hochdotiertem Diamant ergibt sich außerdem daraus, daß der elektrische Widerstand dieses Materials weitgehend temperaturunabhängig ist. Zwar zeigen andere Halbleiterstoffe mit einem großen Bandabstand auch weitgehende Temperaturunabhängigkeit im Temperaturbereich der Anwendung (z.B. SiC), aber nur Diamant ist gleichzeitig auch chemisch inert.

Ein weiterer großer Vorteil der erfindungsgemäßen diamantenen Kontaktbereiche ergibt sich daraus, daß eventuell doch entstandene Anhaftungen am Diamant relativ einfach entfernt werden können. Hierbei zeigt Diamant eine gute Widerstandsfähigkeit gegen mechanische und aggressive chemische Reinigung. Außerdem ist es mit dem erfindungsgemäßen Verfahren möglich, die Kontakte elektrolytisch zu reinigen, ohne daß es zu einer Schädigung des Elektrodenmaterials kommt. Somit ist das erfindungsgemäße Instrument oft wiederverwendbar und dadurch wirtschaftlich und umweltfreundlich. Wegen der möglichen Wiederverwendbarkeit kann das Instrument auch an anderen Stellen hochwertige Ausführungsformen annehmen, welche für den Operateur eine Verbesserung darstellen, ohne daß es zu wirtschaftlichen Nachteilen kommt.

Vorteilhafte Ausgestaltungen der vorliegenden Erfindung werden in den abhängigen Ansprüchen angegeben.

Ein besonders großer Vorteil ergibt sich daraus, daß der Kontaktbereich aus dotiertem Diamant einen hohen Bandabstand zwischen 5,2 eV und 5,6 eV, vorzugsweise zwischen 5,3 eV und 5,5 eV, besonders vorzugsweise von 5,45 eV (eV = Elektronenvolt) besitzt. Dies ist für die spätere elektrochemische Reinigung des Instrumentes von größter Bedeutung. Dies liegt daran, daß es zur Reinigung eines verschmutzten Kontaktbereiches möglich ist, an diesen eine Spannung anzulegen, ohne daß es zu einer Zersetzung des Kontaktbereiches kommt. Bei diesen hohen Spannungen werden andererseits selbst bei destilliertem Wasser OH⁻-Ionen und Ozon in großer Menge gebildet. Hierdurch wird das Medium so aggressiv, daß selbst organische Bestandteile wie zum Beispiel Prionen rückstandsfrei vom Kontaktbereich gelöst und vernichtet werden.

Eine vorteilhafte Ausgestaltung sieht vor, daß die Kontaktbereiche einen elektrisch leitenden Kern aufweisen. Dieser Kern, welcher vorzugsweise aus einem (Hart-)Metall oder aus den in der Medizin oft verwendeten Materialien wie Niob, Iridium, Tantal, Wolfram oder Titan besteht, ist einerseits mechanisch stark beanspruchbar und stellt andererseits einen guten elektrischen Leiter dar, welcher die Verbindung zu dem Kontaktbereich herstellt. Der Kern kann auch aus Graphit, Zr oder kohlefaserverstärktem Kohlenstoff bestehen. Die Abscheidung von Diamant aus einem Plasma führt zu einer chemischen Bindung zwischen dem Kernmaterial und der Diamantschicht. Dadurch entsteht eine sehr feste Verbindung von Kernmaterial und Kontaktbereich. Hierbei werden bevorzugt CVD (Chemical Vapor Deposition)-Verfahren angewandt, die zur Beschichtung dreidimensionaler Werkstoffe besonders geeignet sind. Hervorzuheben ist hier die Hot-Filament-CVD. Diese ist sehr flexibel bezüglich der Form der zu beschichtenden Bauteile (und ist damit selbst für die Beschichtung von Bohrern verwendbar). Neben Hot-Filament-CVD sind noch Mikrowellen-Plasma-CVD und ECR (Electron-Cyclotron-Resonance) unterstützte Mikrowellen-CVD möglich. Gemeinsame Vorteile dieser Verfahren sind die gute, homogene Beschichtung von dreidimensionalen Bauteilen und die hohe Flexibilität bezüglich der beschichtbaren Formen.

Es sind jedoch auch andere Ausführungsformen von Instrumenten vorteilhaft, welche keinen elektrisch leitenden Kern vorsehen. So ist es z.B. möglich, die Schenkel des erfindungsgemäßen Instruments im Kern aus einem nicht elektrisch leitfähigem Material herzustellen und diesen Kern auf voller Länge, d.h. von der Koagulationsspitze bis zum Anschlußkontakt für die Spannungsversorgung mit einer elektrisch leitfähigen Schicht, z.B. der erfindungsgemäßen dotierten Diamantschicht, zu beschichten. Auf diese Beschichtung kann dann bei Bedarf bereichsweise ein Isoliermaterial aufgebracht werden.

Darüber hinaus sind noch weitere erfindungsgemäße Ausführungsformen möglich, etwa ein elektrisch leitender bzw. nichtleitender Diamantkern. Ferner ist der Auftrag von Zwischenschichten zwischen einem beliebigen Kern sowie der erfindungsgemäßen dotierten Diamantschicht möglich. Eine Ausführungsform sieht vor, daß auf der leitfähig dotierten Diamantschicht des Kontaktbereiches zumindest bereichsweise weitere leitfähige Schichten aufgebracht sind. In diesem Fall dient der Diamant zur Vermeidung von Hot Spots durch verbesserte Wärmeverteilung im Kontaktbereich.

Die Dotierstoffkonzentration im Kontaktbereich beträgt mehr als 5 x 10¹⁸ cm⁻³. Hierbei sind als besonders vorteilhafte Dotierstoffe Bor, Schwefel, Lithium oder Titan zu nennen, möglich sind z.B. auch Stickstoff, Phosphor oder sp²-gebundener Kohlenstoff in der Diamantschicht. Die eingangs geschilderten erfindungsgemäßen Vorteile ergeben sich hierbei in besonderer Weise dadurch, daß der spezifische Widerstand des dotierten Diamanten der Kontaktbereiche kleiner als 100 Ohm cm ist, bevorzugt kleiner als 1 Ohm cm, besonders bevorzugt kleiner als 0,01 Ohm cm, während die mittlere Schichtdicke des Kontaktbereiches bis zu 300 Mikrometer, bevorzugt weniger als 10 Mikrometer, besonders bevorzugt 1 bis 5 Mikrometer beträgt. Mit diesen Parametern wird gewährleistet, daß der Serienwiderstand der Beschichtung klein ist, so daß sich eine vernachlässigbare Eigenerwärmung ergibt, die Eigenwärme wird außerdem durch die hohe Wärmeleitfähigkeit und geringe Wärmekapazität schnell an die Umgebung abgegeben.

Eine besonders vorteilhafte Ausgestaltung ergibt sich daraus, daß das chirurgische Instrument mit Steuergeräten nach dem Stand der Technik betreibbar ist (d.h. es ist keine teure Neuanschaffung eines Steuergeräts nötig). Das Instrument ist aber auch mit einem erfindungsgemäßen speziellen Steuergerät elektrisch verbindbar, welches nur eine sehr kleine Abwandlung von solchen nach dem Stand der Technik darstellt (dieses ist ergänzt um eine "Reinigungsoption", d.h. daß eine Spannnungsquelle vorgesehen ist, welche bei der anschließenden elektrochemischen Reinigung zur Spannungsversorgung dient). Beide Steuergeräte weisen jedenfalls einen Hochfrequenz-Generator zur Erzeugung unmodulierter und modulierter Hochfrequenzströme durch die Kontaktbereiche hindurch auf.

Besonders vorteilhaft ist jedoch, daß das erfindungsgemäße Steuergerät ein mit Flüssigkeit füllbares Tauchbecken zum Eintauchen der Kontaktbereich in diese Flüssigkeit und eine Spannungsquelle zum Anlegen einer Gleich- oder Wechselspannung an die in die Flüssigkeit eingetauchten Kontaktbereiche aufweist bzw. mit dem Tauchbecken/der Spannungsquelle verbindbar ist. Hierdurch kann, gegebenenfalls sogar ohne mechanische Grobvorreinigung, der diamantene Kontaktbereich von Anhaftungen elektrochemisch gereinigt werden. Dies kann, je nach Wahl der weiteren Verfahrensparameter, so gründlich erfolgen, daß unter Umständen eine nachfolgende zusätzliche z.B. Dampfsterilisation entfallen kann (dies ist in Deutschland derzeit allerdings noch gesetzlich vorgeschrieben).

Es ist hierbei besonders vorteilhaft, wenn die von der Spannungsquelle erzeugte Spannung eine Amplitude zwischen 0 und 1000 V, vorzugsweise 0 bis 10 V, besonders vorzugsweise 1 bis 5 V beträgt (der Spannungsbereich bis zu 5,45 Volt zeigt den besonderen Vorteil, daß aus dem Wasser heraus aggressive Hydroxidionen und Ozon gebildet wird zum Abtrag von Verschmutzungen und der Desinfizierung der Kontaktbereiche, ohne daß es zu einer Zersetzung des Kontaktbereiches kommt). Hierbei beträgt die Stromdichte an den Außenflächen der dotierten Diamantkontaktbereiche vorteilhafterweise bis zu 10 A pro cm².

Die elektrochemische Reinigung, welche bei in einer destilliertes Wasser enthaltenden Flüssigkeit getauchten Instrumentenschenkeln erfolgt (hierbei sind, wenn zwei Schenkel einer Pinzette eingetaucht sind, die Kontaktbereiche der beiden Schenkel im festen Abstand zueinander angeordnet) wird dadurch verbessert, daß zusätzlich die Flüssigkeit mit Zusätzen, wie etwa Lösungsmitteln, Reinigungsmitteln, Desinfektionsmitteln, Säuren (z.B. Schwefelsäure) und anderen, auch festen/löslichen Zusätzen bzw. Zusätzen, die die eletrische Leitfähigkeit verursachen, versetzt wird. Zusätzlich kann die Flüssigkeit auch erwärmt bzw. mit Ultraschall beaufschlagt werden, um die Reinigungswirkung zu beschleunigen bzw. zu verbessern.

Eine weitere vorteilhafte Ausgestaltung des Instruments sieht vor, daß die Kontaktbereiche z.B. auf den jeweiligen Schenkeln einer Pinzette eine gleichgerichtete oder eine entgegengesetzte Polung aufweisen und in entsprechender Weise so an das Steuergerät angeschlossen bzw. angesprochen werden. Somit ist es möglich, sowohl im Operationsbetrieb als auch bei der anschließenden elektrochemischen Reinigung wahlweise monopolar oder bipolar vorzugehen, hierbei kann die Polung desselben Instruments im Koagulations- und Reinigungsbetrieb unterschiedlich sein.

Weitere vorteilhafte Ausgestaltungen der vorliegenden Erfindung werden in den übrigen abhängigen Ansprüchen gezeigt.

Die vorliegende Erfindung wird nun anhand mehrerer Figuren erläutert. Es zeigen:
- Fig. 1: eine erfindungsgemäße Pinzette, welche an ein Steuergerät angeschlossen ist,
- Fign. 2a bis 2c: Schnitte durch die Schenkelspitzen einer erfindungsgemäßen Pinzette,
- Fign 3a bis 3e: Schnitte durch die Schenkelspitzen einer weiteren Ausführungsform einer erfindungsgemäßen Pinzette,
- Fign. 4a bis 4c: Schnitte durch die Schenkelspitzen nach einer weiteren Ausführungsform einer erfindungsgemäßen Pinzette,
- Fign. 4d bis 4g: Schnitte durch die Schenkelspitzen weiteren Ausführungsformen erfindungsgemäßer chirurgischer Instrumente,
- Fign. 5a und 5b: die prinzipielle Verschaltung einer erfindungsgemäßen Pinzette im bi- und monopolaren Betrieb,
- Fign. 6a und 6b: Darstellungen des Stromflusses im mono- und bipolaren Betrieb,
- Fign. 7a und 7b: die Reinigung einer erfindungsgemäßen Pinzette im bi- und monopolaren Betrieb, und
- Fig. 8: Elektrolyse-Strom-Spannungskurve für eine erfindungsgemäße Pinzette.

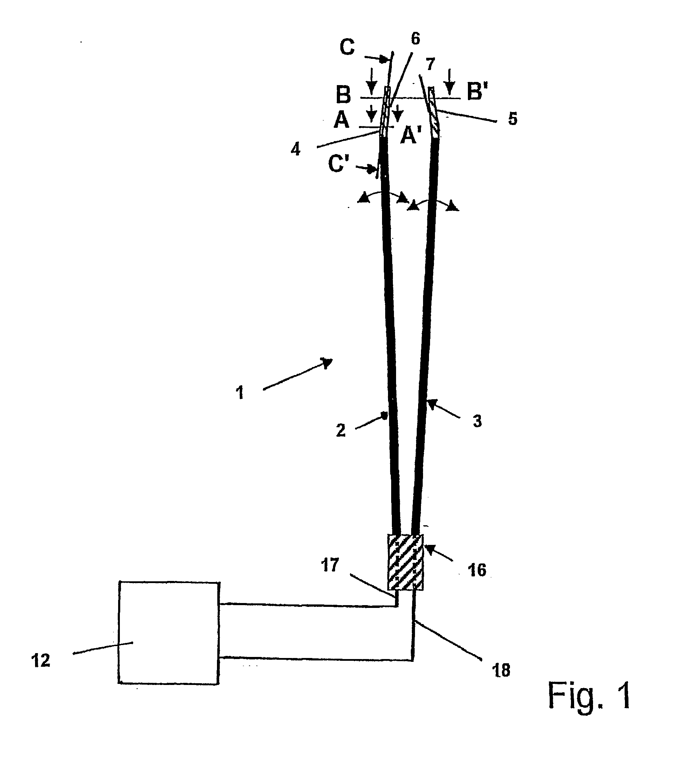

Fig. 1 zeigt eine Pinzette 1 für chirurgische Zwecke, insbesondere zur Koagulation als ein Beispiel für ein chirurgisches Instrument. Diese weist zwei zueinander bewegbare elektrisch gegeneinander isolierte Schenkel 2 und 3 auf. Der Schenkel 2 weist an seiner Spitze 4 (bereichsweise) einen elektrischen Kontaktbereich 6 auf, welcher aus elektrisch leitfähigen dotierten Diamant besteht. Der gegenüberliegende Schenkel 3 weist entsprechend an der Spitze 5 einen Kontaktbereich 7 aus dem zur Herstellung der elektrischen Leitfähigkeit dotierten Diamant auf.

Der elektrische Kontaktbereich 6 ist durch den Schenkel 2 hindurch über eine elektrische Verbindung 17 mit dem Steuergerät 12 verbunden. Entsprechend ist der Kontaktbereich 7 des Schenkels 3 über eine elektrische Verbindung 18 mit dem Steuergerät 12 verbunden. Der Träger 16 sowie die darin gehaltenen Schenkel 2 und 3 haben in dem von den Schenkelspitzen verschiedenen Bereich eine Umhüllung oder dergleichen zur elektrischen Isolation des Schenkels 2 vom Schenkel 3.

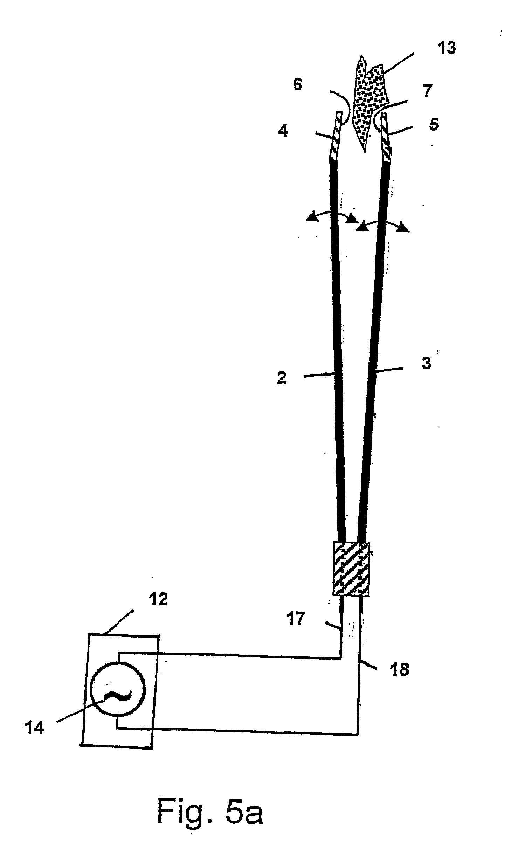

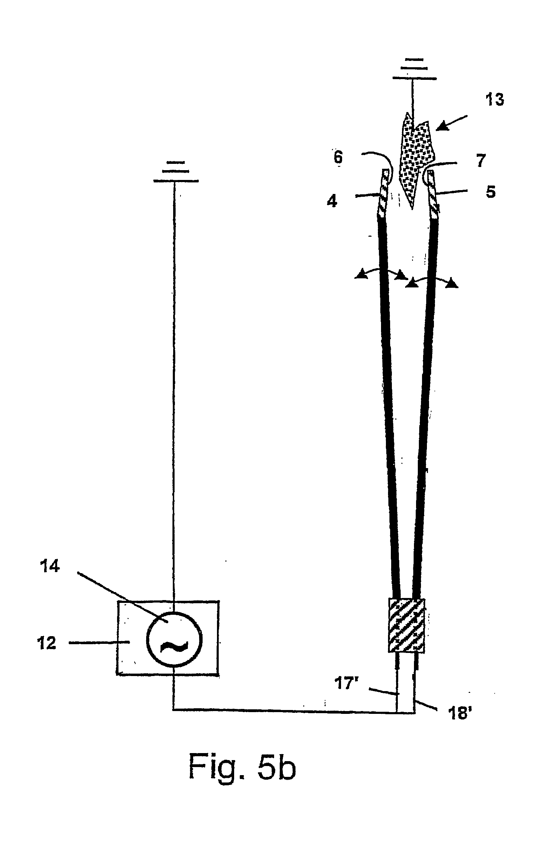

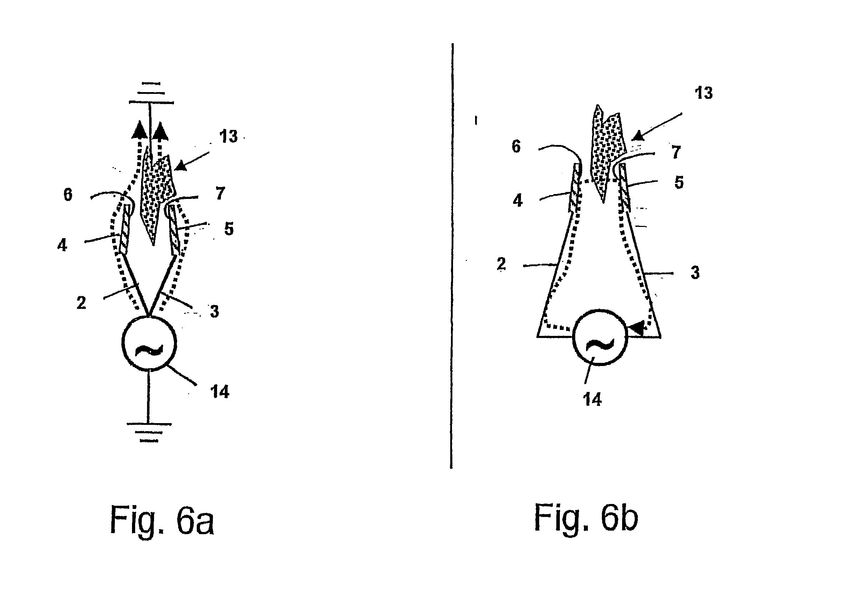

Die Schenkel 2 und 3 sind zueinander bewegbar, so daß zwischen den Schenkelspitzen 4 und 5 ein nicht dargestelltes Stück Gewebe erfaßt und aufgrund eines im Steuergerät 12 erzeugten Stromflusses zum Zwecke der Koagulation elektrisch durchströmt werden kann. Auf genauere Einzelheiten der Koagulation wird später (insbesondere bei der Beschreibung der Fign. 5a, 5b, 6a, 6b) näher eingegangen.

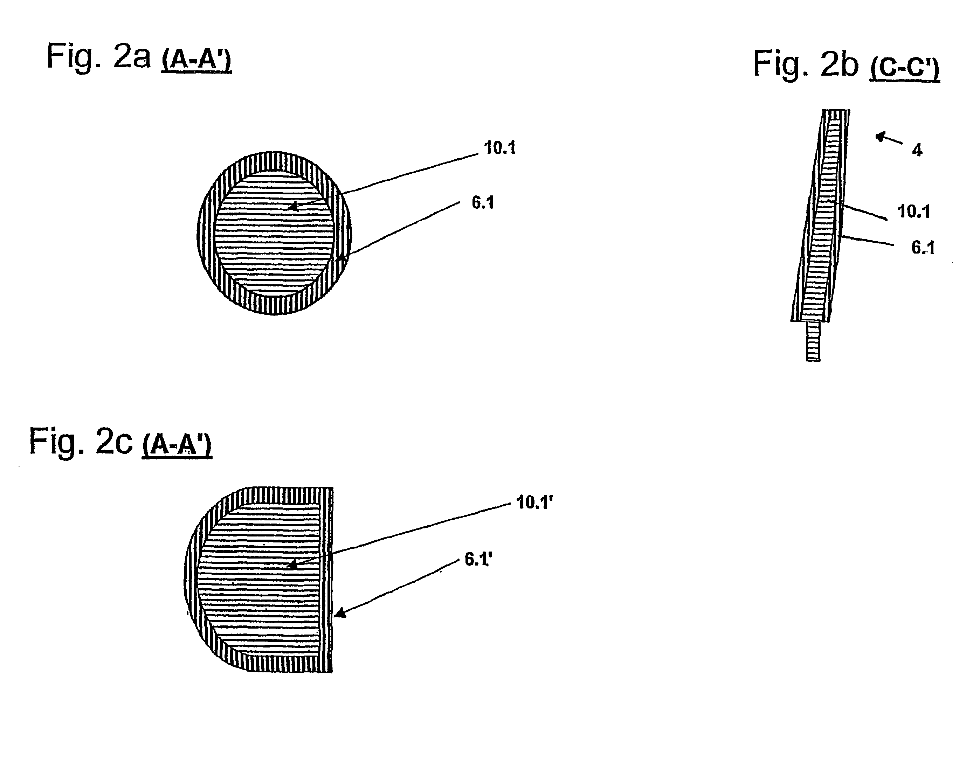

Fign. 2a bis 2c zeigen Schnitte durch die Spitze 4 des Schenkels 2 (für die Schenkelspitze 5 ergeben sich identische, jedoch spiegelverkehrte Ansichten, Analoges gilt für die Zeichnungen 3a bis 3d sowie 4a bis 4c).

Fig. 2a zeigt einen Schnitt gemäß A-A' durch die Spitze 4 des Schenkels 2. Die Schenkelspitze 4 enthält einen elektrisch leitenden Kern 10.1. Dieser Kern 10.1 ist mit dem Steuergerät 12 über den Schenkel 2 bzw. die Zuleitung 17 elektrisch verbunden (Entsprechendes ergibt sich selbstverständlich auch für den Schenkel 3). Auf den Kern 10.1 ist das den Kontaktbereich 6.1 bildende dotierte Diamantmaterial aufgebracht. In der vorliegenden Ausführungsform ist das den Kontaktbereich bildende dotierte Diamantmaterial konzentrisch um den im Querschnitt runden Kern 10.1 angeordnet. Der Kern ist bevorzugt aus einem refraktären Metall, bevorzugt Titan, Wolfram, Niob, Tantal oder Legierungen dieser Metalle. Es sind jedoch auch andere Substratmaterialien (Silizium, Siliziumcarbid, Graphit, Karbide refraktärer Metalle, Iridium oder andere Materialien mit geringer Gitterfehlanpassung, Halbleiter (z.B. Ge) oder damit beschichtete andere Kernmaterialien erfindungsgemäß möglich. Der Kontaktbereich 6.1 aus dotiertem Diamantmaterial ist mittels eines CVD-Verfahrens auf den Kern 10.1 aufgebracht. Hierfür eignen sich insbesondere CVD-Verfahren, welche einen gleichmäßigen Auftrag der den Kontaktbereich bildenden diamantenen Schicht auf dreidimensionalen Gebilde ermöglicht (siehe oben). Der Halt zwischen der den Kontaktbereich 6.1 bildenden Diamantschicht und dem Kern 10.1 ist besonders fest, dies ergibt sich daraus, daß zwischen Kern 10.1 und Kontaktbereich 6 eine chemische Bindung besteht. Die Dotierstoffkonzentration des Kontaktbereiches beträgt mehr als 5 x 10¹⁸ cm⁻³, im vorliegenden Falle hat sich eine Dotierstoffkonzentration des Kontaktbereiches im Bereich von 5 x 10²⁰ cm⁻³ besonders bewährt. Als bevorzugte Dotierstoffe des dotierten Diamantmaterials des Kontaktbereiches kommen hierbei Bor, Schwefel, Lithium oder Titan in Betracht, möglich sind z.B. auch Stickstoff, Phosphor oder sp²-gebundener Kohlenstoff in der Diamantschicht. Der Auftrag bzw. die Dotierung der diamantenen leitenden Schicht erfolgte vorliegend so, daß keine Diffusion der Dotierstoffe aus dem Diamant geschieht. Die Diamantschicht kann zur Herstellung einer kleineren Oberflächenrauhigkeit aus texturiertem oder nanokristallinen Diamant sein.

Es sind somit vielfältige Diamantbeschichtungen herstellbar. Zur leichten Unterscheidbarkeit/Kennzeichnung ist es möglich, die Interferenzfarben der Diamantbeschichtung zu nutzen.

Der spezifische Widerstand des den Kontaktbereich bildenden dotierten Diamantmaterials ist über die Dotierstoffkonzentration einstellbar. Als vorteilhaft haben sich erwiesen spezifische Widerstände kleiner als 100 Ohm cm, bevorzugt kleiner als 1 Ohm cm, im vorliegenden Falle beträgt er 0,01 Ohm cm. Hierbei beträgt die Schichtdicke der auf den Kern 10.1 aufgetragenen diamantenen Schicht vorzugsweise weniger als 300 Mikrometer, bevorzugt weniger als 10 Mikrometer. Im vorliegenden Falle beträgt er besonders vorzugsweise zwischen 1 und 5 Mikrometer.

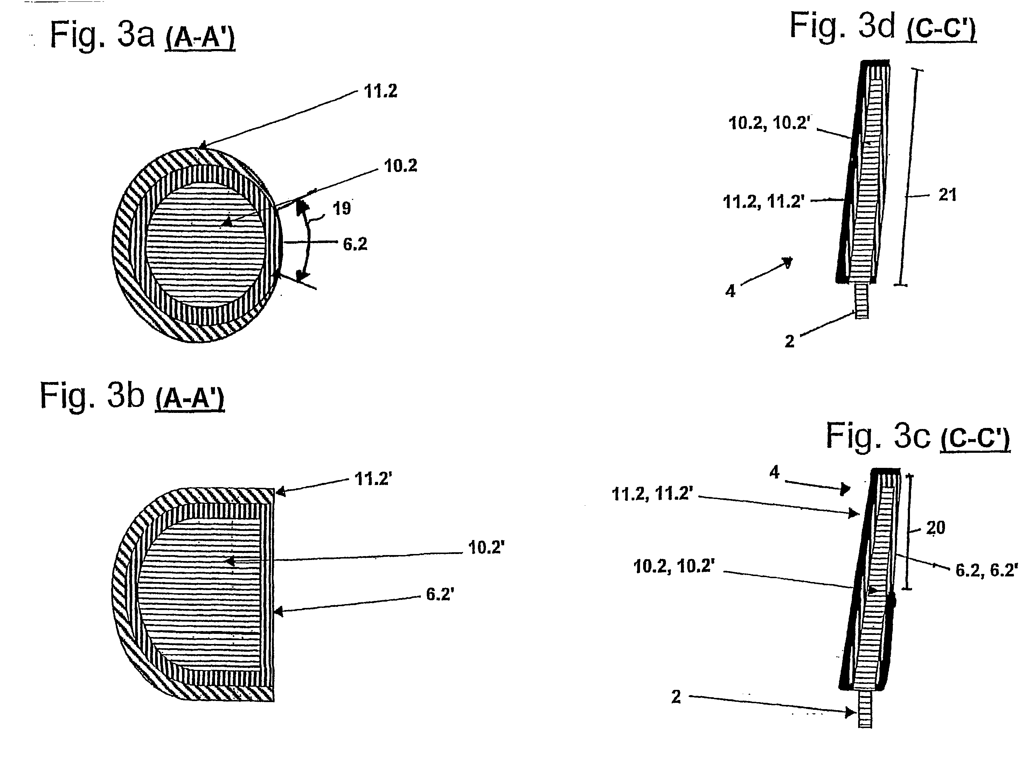

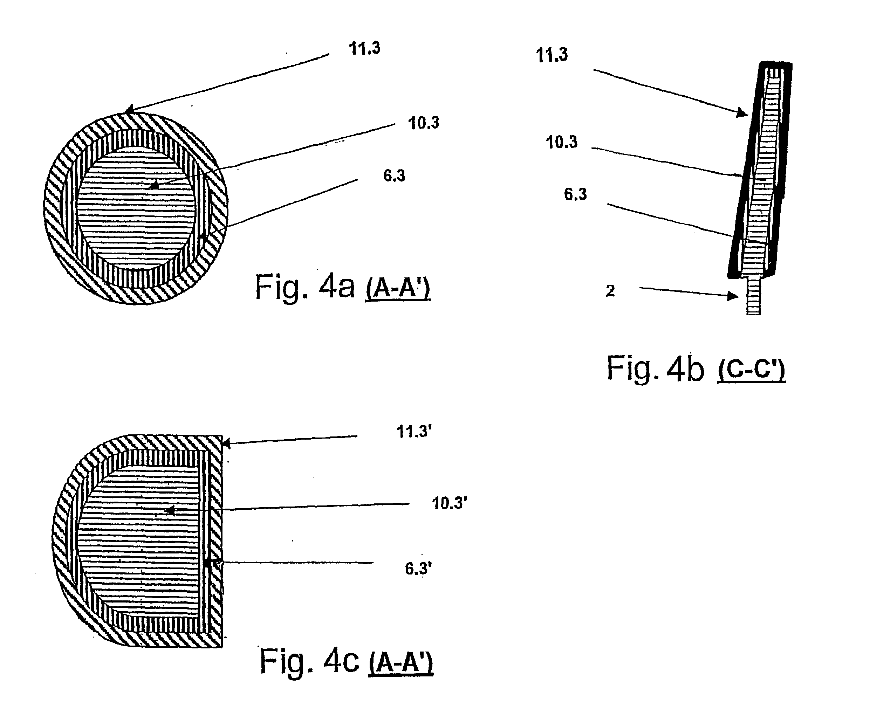

Die grundlegenden Eigenschaften des den Kontaktbereich bildenden Diamant bzw. des Kerns wurden soeben anhand von Fig. 2a erläutert. Es wird nachdrücklich betont, daß sämtliche Angaben bezüglich des Kernmaterials, der Auftragsweise der leitenden diamantenen Schicht auf den Kern, der Dotierstoffe des Diamantmaterials des Kontaktbereiches, der Dotierstoffkonzentration, der Schichtdicken und der spezifischen Widerstände auch direkt für die Ausführungsformen nach Fign. 3a bis 3e bzw. 4a bis 4g gelten, sofern dort nichts anderes ausdrücklich gesagt ist.

Fig. 2b zeigt einen Längsschnitt gemäß C-C' aus Fig. 1. Hierin ist zu sehen, wie die den Kontaktbereich 6.1 bildende dotierte Diamantschicht "hutartig" auf den Kern 10.1 aufgebracht ist (es ist jedoch auch eine komplett umschließende Beschichtung möglich). Außerdem ist der Montagebereich der Spitze 4 des Schenkels zu sehen. Dieser Montagebereich befindet sich an der unteren Kante der Spitze, welche dann sprungartig in den Steg des übrigen Schenkels übergeht. Eine besondere Ausführungsform der Erfindung sieht zur kostengünstigen Herstellung einer erfindungsgemäßen Pinzette vor, daß lediglich die Spitze 4 (d.h. der Pinzettenkopf) aus diamantbeschichtetem Material (zum Beispiel einem Material wie einem refraktionären Metall, bevorzugt Titan, Wolfram, Niob, Tantal oder Legierungen dieser Metalle oder aus Silizium, Siliziumkarbid, Graphit, Karbiden refraktionärer Metalle, Iridium oder anderen Materialien mit geringer Gitterfehlanpassung, Halbleiter (z.B. Ge) oder damit beschichteten anderen Kernmaterialien) besteht. Der übrige Schenkel der Pinzette kann aus einem kostengünstigen Material wie zum Beispiel Edelstahl gefertigt sein. Der fertig beschichtete Pinzettenkopf, welcher an seiner Unterseite zum Beispiel eine Ausnehmung aufweist, wird anschließend auf den übrigen Schenkel aufgeklebt bzw. mit diesem verlötet. Neben den verminderten Herstellungskosten dieser Ausführungsform kann hierdurch erreicht werden, daß eine spezifische Koagulation nur an ganz definierten Stellen der Pinzette vorgenommen wird. So ist es z.B. auch möglich, den Schenkel (bis auf die Spitze 4) aus Kunststoff mit innenliegenden elektrischen Zuleitungen herzustellen (damit ist durch den Kunststoff einerseits eine elektrische Isolation gegeben und andererseits der Stromfluß nur durch die Spitze möglich). Es soll hervorgehoben werden, daß diese kostengünstige Herstellungsmethode nicht nur für die in Fign. 2a bis 2c gezeigte Ausführungsform gelten soll, sondern auch für sämtliche weiteren Ausführungsformen des hier beschriebenen chirurgischen Instrumentes.

Fig. 2c zeigt eine weitere Ausführungsform eines Schnittes gemäß A-A' aus Fig. 1, welche sich lediglich von der in Fig. 2a gezeigten Version dadurch unterscheidet, daß an der dem Schenkel 3 zugewandten Innenseite die Spitze 4 des Schenkels 2 abgeflacht ist. Ansonsten gilt hier das zu Fig. 2a Gesagte.

Fign. 3a bis 3e zeigen eine weitere Variante der Ausführung von Schenkelspitzen 4 bzw. 5 nach dem Grundprinzip der in Fig. 1 gezeigten Pinzette.

Fig. 3a zeigt einen Schnitt gemäß A-A' durch eine Schenkelspitze 4. Hierbei entsprechen Aufbau und Geometrie des Kerns 10.2 sowie der den Kontaktbereich 6.2 bildenden diamantenen leitenden Schicht im Wesentlichen der in Fig. 2a gezeigten Ausführungsform.

Zusätzlich ist jedoch die den Kontaktbereich 6.2 bildende diamantene leitfähige Schicht mit einer im Wesentlichen sichelartig geformten Isolierschicht 11.2 umgeben. Diese Isolierschicht 11.2 ist im Wesentlichen konzentrisch zu der den Kontaktbereich 6.2 bildenden leitfähigen Diamantschicht angeordnet, wobei sich jedoch zu der dem entgegengesetzten Schenkel 3 hingewandten Innenseite hin sich die Dicke der Isolierschicht 11.2 verringert. Diese Verringerung geht so weit, daß in dem von in Fig. 3a eingezeichneten Marken 19 eingegrenzten Winkelbereich der Kontaktbereich 6.2 vollkommen freiliegt, d.h. nicht von der Isolierschicht 11.2 bedeckt ist.

Das Material der Isolierschicht 11.2 ist nominell undotierter Diamant, d.h. elektrisch nicht leitfähiger Diamant. Die Isolierschicht 11.2 wurde in einem CVD-Verfahren auf die den Kontaktbereich 6.2 bildende leitende Diamantschicht aufgebracht. Hierzu eignet sich wiederum die oben bezeichneten CVD-Verfahren. Die Isolierschicht kann jedoch auch neben Ausführungsformen aus texturiertem Diamant oder nanokristallinem Diamant (dies ist wegen der geringen Rauheit der Oberfläche besonders günstig) auch aus DLC (Diamond like Carbon) bestehen. Diese elektrisch isolierende Schicht 11.2 kann hierbei wiederum verschieden terminiert werden, um das Anhaften von Gewebe noch besser zu verhindern (siehe hierzu die Ausführungen zur Terminierung in der Beschreibungseinleitung, welche entsprechend anwendbar sind).

Fig. 3b zeigt eine Variante des Schnittes A-A'. Hierbei ist der Aufbau des Kerns bzw. der leitenden Diamantschicht entsprechend der Variante aus Fig. 2c, zusätzlich wurde, abgesehen von dem Bereich der abgeflachten Innenseite, die leitende Diamantschicht mit einer Isolierschicht 11.2' versehen.

Fign. 3c und 3d zeigen zwei Varianten eines Längsschnittes gemäß C-C' durch in Fig. 3a bzw. 3b gezeigte Schenkelspitzen 4. Hierbei entspricht die Grundform jeweils der in Fig. 2b gezeigten Variante. Diese wird ergänzt durch die aufgebrachte Isolierschicht 11.2 bzw. 11.2'. In Fig. 3c ist die den Kontaktbereich 6.2 bzw. 6.2' bildende leitende Diamantschicht im Wesentlichen umlaufend mit der isolierenden Diamantschicht 11.2 bzw. 11.2' bedeckt, wobei lediglich auf der Innenseite der Schenkelspitze 4, etwa im Bereich der oberen Hälfte 20, die Isolierschicht ausgespart ist, so daß es hier zur elektrischen Kontaktierung kommen kann.

Diese Ausführungen gelten für die Ausführungsform nach Fig. 3d im Wesentlichen entsprechend, allerdings ist hier die Innenseite auf der gesamten Höhe der Schenkelspitze 4, d.h. im Bereich 21, von der Isolierschicht 11.2 bzw. 11.2' nicht bedeckt.

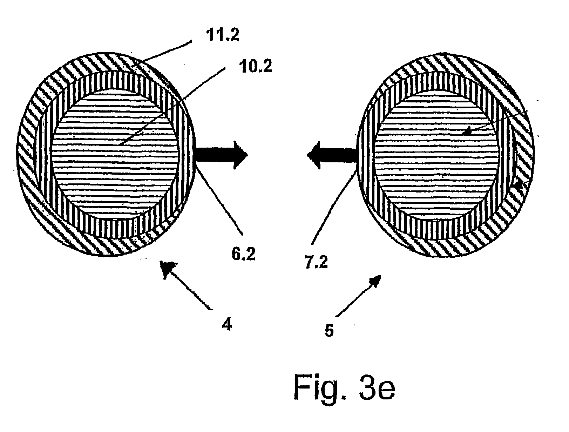

Schließlich zeigt Fig. 3e einen Schnitt B-B' durch zwei Schenkelspitzen 4 und 5, welche der Ausführungsform aus Fig. 3a entsprechen. Die Ausbildung der Spitze 5 ist im Wesentlichen spiegelsymmetrisch zum oben beschriebenen Aufbau der Schenkelspitze 4. Die elektrischen Kontaktbereiche aus dotiertem Diamantmaterial 6.2 bzw. 7.2, welche nicht von der Isolierschicht 11.2 bedeckt sind, stehen sich direkt gegenüber und können durch Bewegen der Schenkel 2 bzw. 3 aufeinander zu bewegt werden, um in ihrem Zwischenraum ein Stück Gewebe zu erfassen und dieses elektrisch zu durchströmen.

Fign. 4a bis 4g zeigen eine weitere Ausführungsform einer erfindungsgemäßen Schenkelspitze 4. Diese entspricht im Grundaufbau wiederum zunächst der in Fig. 2a bis 2c gezeigten Schenkelspitze. Allerdings ist hier die Isolierschicht 11.3 (bezüglich Material und Auftragsweise dieser Schicht wird ausdrücklich auf die Beschreibung der Fign. 3a bis 3e verwiesen) so gestaltet, daß die leitfähig dotierte Diamantschicht vollkommen durch die Isolierschicht 11.3 elektrisch isoliert wird. Diese Ausführungsform erlaubt eine kapazitive Leistungsübertragung auf das Gewebe bei elektrischer Beaufschlagung des isolierten Kontaktbereiches.

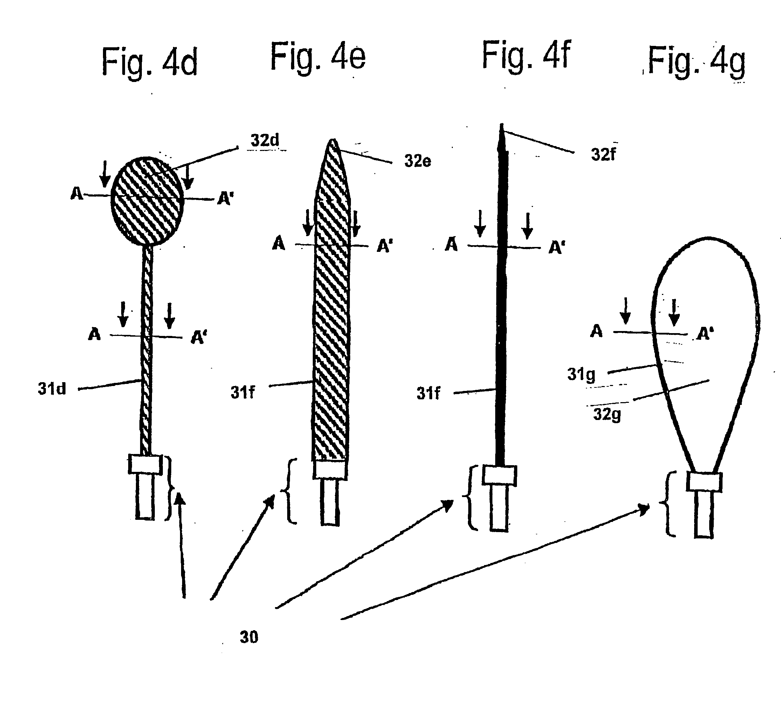

Fign. 4d bis 4g zeigen mehrere Ausführungsformen monopolarer Koagulationsinstrumente. Diese weisen unterschiedliche Schenkel 31d bis 31g auf, an die sich jeweils identisch ausgeführte Steckadapter 30 anschließen. Die Koagulation erfolgt jeweils durch Kontakt der Schenkelspitze mit dem zu koagulierenden Gewebe des Patienten, als Gegenelektrode dient z.B. ein Operationstisch, auf welchem sich der Patient befindet.

Der Schenkel 31d ist im wesentlichen stabförmig ausgeführt, an der Spitze des Stabes schließt sich eine im Wesentlichen runde Kugelelektrode 32d an.

Der Schenkel 31e ist im wesentlichen "schwertförmig" ausgeführt, d.h. in der Blattebene flach im Vergleich mit der hier dargestellten Draufsicht und an der Spitze 32e spitz zulaufend.

Der Schenkel 31f ist im wesentlichen nadelförmig ausgeführt, d.h. langestreckt mit vergleichsweise scharfer Spitze 32f.

Der Schenkel 31g ist im wesentlichen schlingenförmig ausgeführt, d.h. als im wesentlichen geschlossene Kurve bzw. Schlaufe mit einem Auge 32g.

Die in Fign. 4d, 4e, 4f, 4g gezeigten Instrumente weisen an den mit A-A' bezeichneten Schnittstellen Querschnitte auf, wie sie bereits in den Fign. 2a, 3a, 4a (bzw. für nichtrunde Querschnitte z.B. 2c, 3b, 4c) eingehend erläutert wurden, so daß auf eine nochmalige Schilderung verzichtet wird. Bezüglich Fig. 4d bis 4g ist zu bemerken, daß auch hier die Schenkel z.B. Schenkel 31e in Fig. 4e Schichten von dotierten bzw. undotierten Diamantmaterialien wie oben beschrieben aufweisen können. Allen Ausführungsformen gemeinsam ist, daß diese an den Schenkelspitzen Kontaktbereiche zur Koagulation durch Stromfluß aufweisen.

Der Steckadapter 30 ist in einen nicht dargestellten Instrumentenhalter steckbar zur Herstellung einer elektrischen Verbindung und mechanischen Fixierung. Dieser Instrumentenhalter entspricht in Aufbau und Funktion zunächst dem Träger 16 aus Fig. 1, d.h. daß er in entsprechender Weise die elektrische Verbindung von einem mit dem Steuergerät 12 verbundenem elektrischen Kontakt zu den Kontaktbereichen der Schenkel herstellt (wobei jedoch nur die monopolare Verschaltung nach Fig. 5b bzw. Fig. 6a zur Anwendung kommt). Zusätzlich weist der Instrumentenhalter jedoch einen Handgriff auf, welcher das Halten des Instrumentenhalters in der Hand des Operateurs ermöglicht.

In den oben beschriebenen Beispielen wurde die Isolierschicht, z.B. die Isolierschicht 11.2 auf Bereiche der elektrisch leitfähigen Diamantschicht aufgebracht. Es ist jedoch selbstverständlich möglich, diese Isolierschicht gegebenenfalls auch auf Bereiche des Kerns, z.B. 10.2, direkt aufzubringen. Dann gelten die oben beschriebenen Figuren 3 und 4 analog.

In Fign. 5a, 5b, 6a, 6b wird die Koagulation von Gewebe mittels der erfindungsgemäßen Pinzette kurz erläutert.

Fig. 5a zeigt einen prinzipiellen Aufbau gemäß Fig. 1. In den Bereich zwischen den Innenseiten der Schenkelspitzen 4 und 5 ragt ein Stück menschliches Gewebe 13 hinein. Das Steuergerät 12 ist mit einem Hochfrequenz-Generator 14 zur Erzeugung unmodulierter und modulierter Hochfrequenzströme durch die an den Innenseiten der Schenkelspitzen 4 und 5 angeordneten elektrischen leitfähigen Kontaktbereiche hindurch ausgestattet. Die Schenkel 2 und 3 sind hierbei zueinander beweglich angeordnet, so daß ein von den Schenkelspitzen 4 und 5 zu erfassendes Gewebestück 13 an den Innenseiten der Schenkelspitzen 4 und 5 festgeklemmt werden kann.

Fig. 5a zeigt den Aufbau der erfindungsgemäßen Pinzette im bipolaren Betrieb (Schenkel 2 und 3 sind in ihrem Träger 16 elektrisch voneinander isoliert). Hierbei weist die Pinzette 2 Pole auf, welche einerseits vom Kontaktbereich 6 über den übrigen Schenkel 2 und die Leitung 17 und andererseits über den Kontaktbereich 7, den übrigen Schenkel 3 und die Leitung 18 zu dem Steuergerät 12 hinführen. Die Koagulation von erfaßtem Gewebe 13 im bipolaren Betrieb ist anhand der Skizze in Fig. 6b abzulesen. Es ist zu sehen, daß der Strompfad bzw. Stromfluß ein geschlossener ist (siehe gestrichelte Linie). Von dem Hochfrequenzgenerator ausgehend, erfolgt der Stromfluß z.B. über den Schenkel 2 und den Kontaktbereich 6 durch das menschliche Gewebe 13 hindurch zu dem Kontaktbereich 7 des Schenkels 3 und wieder zurück zu dem Hochfrequenzgenerator. Es wird also lediglich das zu koagulierende Gebiet in einem sehr begrenzten Raum dem Stromfluß ausgesetzt. Eine noch exaktere Gewebehandlung ist bei Verwendung einer teilweise isolierten Pinzette z.B. nach Fign. 3b, c möglich. Dann kommt es lediglich zwischen den Innenflächen der Spitzen zu einem Stromfluß, umliegendes Gewebe wird nicht beschädigt.

Alternativ ist jedoch auch der monopolare Betrieb möglich (bei Fign. 4d bis 4g ausschließlich).

Fig. 5b zeigt hierzu die prinzipielle Verschaltung der erfindungsgemäßen Pinzette. Hierbei werden die elektrischen Leitungen 17' und 18', welche elektrisch mit den Kontaktbereichen 6 und 7 verbunden sind, zu einer einzigen Leitung elektrisch zusammengefaßt, welche dann in den Hochfrequenzgenerator 14 des Steuergerätes 12 mündet. Der Hochfrequenzgenerator 14 ist hierzu andererseits geerdet. Das zu koagulierende menschliche oder tierische Gewebe 13 ist ebenfalls geerdet, hierzu sind besondere Vorkehrungen zu treffen, um den z.B. auf einem Operationstisch liegenden Menschen/Tiere zu erden. Hier könnten die Leitungen 17'und 18'auch zu einem einzigen Teil zusammengefaßt werden.

Fig. 6a zeigt eine Prinzipskizze des Strompfades bzw. des Stromflusses bei monopolarem Betrieb der erfindungsgemäßen Pinzette wie in Fig. 5b gezeigt. Hierbei fließt der Strom von dem Hochfrequenzgenerator ausgehend durch die elektrisch gleich gepolten Kontaktbereiche 6 und 7 und durch das von der Pinzette erfaßte bzw. die Kontaktbereiche berührte zu koagulierende Gewebe hindurch, z.B. in den Operationstisch. Dieser geerdete Operationstisch enthält eine entsprechend "Neutralelektrode", welche wiederum andererseits mit dem Hochfrequenzgenerator in Verbindung steht. Der Stromfluß ist in Fig. 6a wiederum durch eine gestrichelte Linie dargestellt.

Für die kapazitive Kopplung (siehe auch Fign. 4a bis 4c) gilt prinzipiell dasselbe, allerdings erfolgt hier kein Netto-Stromfluß sondern lediglich ein Wärmefluß durch kapazitiven Energieübertrag.

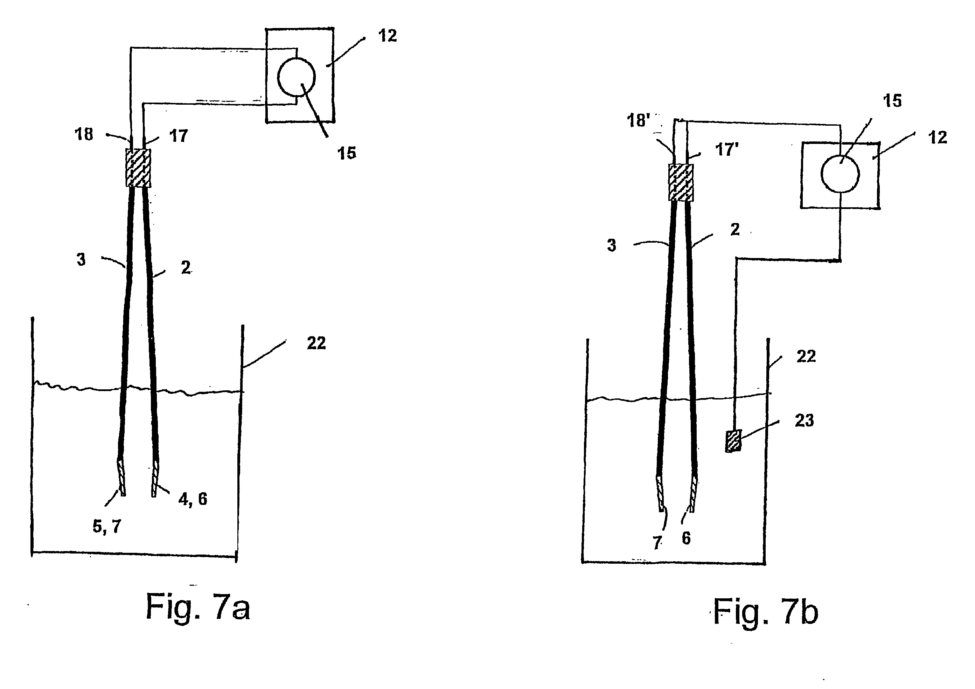

Fign. 7a und 7b zeigen die elektrochemische Reinigung einer mit Gewebeanhaftungen versehenen erfindungsgemäßen Pinzette.

Fig. 7a zeigt ein mit Flüssigkeit füllbares Tauchbekken 22, welches zum Steuergerät 12 gehört bzw. mit diesem verbindbar ist. Das Tauchbecken ist im vorliegenden Fall vorzugsweise mit destilliertem Wasser gefüllt, welchem z.B. Schwefelsäure zur Einstellung der elektrischen Leitfähigkeit zugesetzt wurde (es sind weitere Zusätze möglich, siehe oben). Die Spitzen 4 und 5 der Schenkel 2 und 3 sind in die Flüssigkeit des Tauchbeckens 22 eingetaucht. Die Pinzette ist bipolar verschaltet, d.h. daß ein Pol der Pinzette vom Kontaktbereich 6 ausgehend über die Zuleitung 17 zum Steuergerät 12 führt, der andere Pol über den Kontaktbereich 7 und die Zuleitung 18 zum Steuergerät 12 (siehe analog Fig. 5a). Das Steuergerät 12 enthält eine Spannungsquelle 15, welche wahlweise Gleich- oder Wechselspannung erzeugen kann. Die von dieser Spannungsquelle erzeugbare Spannung liegt bei 0 bis 1000 V, vorzugsweise 0 bis 10 V, besonders vorzugsweise 1 bis 5 V. Es werden Stromdichten an den mit Gewebeanhaftungen versehenen Kontaktbereichen 6 und 7 von bis zu 10 Ampere pro cm² erreicht.

Bei dieser elektrochemischen Reinigung der Kontaktbereiche bzw. der Schenkelspitzen 4 und 5 zeigen sich besonders vorteilhafte Eigenschaften von elektrisch leitfähigem Diamant. Durch den Stromfluß verändert sich der Kontaktbereich aus Diamant nicht, es kommt nicht zu einer möglichen Zersetzung, wie dies bei Metallelektroden der Fall wäre. Aber auch bei der Vorreinigung zeigt der Diamant besonders günstige Eigenschaften, da die mechanische bzw. chemische Vorreinigung aufgrund dr geringeren Haftneigung am Diamant sanfter erfolgen kann. Zudem wären die DiamantKontaktbereiche auch mit hekömmlichen Verfahren reinigbar und dabei robuster als Metallkontakte.

Bei Anlegen einer Spannung an die Kontakte 17 und 18 kommt es zu einer "Selbstreinigung" der diamantenen Kontaktbereiche. Hierbei hat sich gezeigt, daß beim Anlegen von Gleichstrom der Pluspol bzw. die Anode besonders gut gereinigt wird. Zur ausreichenden Reinigung beider Kontaktbereiche bei der Reinigung im hier gezeigten Bipolarbetrieb ist es also notwendig, nach einer gewissen Zeit eine Umpolung vorzunehmen. Dies kann z.B. auch durch Verwendung einer Wechselspannung erreicht werden.

Fig. 7b zeigt die Reinigung einer erfindungsgemäßen Pinzette im monopolaren Betrieb (die Reinigung anderer chirurgischer Instrumente im Sinne der Erfindung, etwa der Koagulationsinstrumente nach Fign. 4d bis 4g erfolgt entsprechend, es ist zu beachten, daß hier jeweils nur ein Schenkel gegeben ist, welcher gegen die Gegenelektrode 23 gepolt wird). Hierbei ist der Aufbau im Wesentlichen identisch zu dem in Fig. 7a beschriebenen, allerdings sind vorliegend die elektrischen Leitungen 18' und 17' zu einem einzigen Pol zusammengefaßt, welcher zu der Spannungsquelle 15 des Steuergerätes 12 führt. Diese Spannungsquelle ist andererseits mit der weiteren Elektrode 23 verbunden, welche ebenfalls in die Flüssigkeit des Tauchbeckens 22 eingeführt ist (in einer weiteren Ausführungsform ist es möglich, die Wandung des Tauchbeckens selbst als Gegenelektrode 23 auszubilden bzw. die Elektrode in dieser Wandung vorzusehen). Bei der hier gezeigten elektrochemischen Reinigung haben somit die Kontaktbereiche 6 und 7 eine gleichgerichtete Polung (siehe auch Fig. 5b). Hier ist folglich die Reinigung beider Spitzen der Pinzette gleichzeitig möglich. Pinzipiell kann sowohl Gleich- als auch Wechselspannung zur Anwendung kommen. Die Reinigung der monopolaren Instrumente nach 4d bis 4g erfolgt analog, hierbei ist der zwei-schenkelige Einsatz in die Fassung 16 durch die Halterung für die Elektroden nach 4d bis 4g ersetzt.

Neben den in Fig. 7a und 7b beschriebenen Reinigungsarten sind noch weitere für die erfindungsgemäßen chirurgischen Instrumente möglich. Bei den oben beschriebenen Varianten wurde die Spannung direkt an die Kontaktbereiche des chirurgischen Instrumentes angelegt ("aktive" elektrochemische Reinigung). Es ist aber auch eine "passive" chemische Reinigung möglich, bei der zwei Tauchbadelektroden, zwischen denen eine Spannung aufgebaut wird, in ein Flüssigkeitsbad getaucht werden und das chirurgische Instrument zur Reinigung in dieses Flüssigkeitsbad, vorzugsweise im Bereich zwischen den Tauchbadelektroden, getaucht wird. Es ist hierbei besonders empfehlenswert, wenn mindestens eine der Tauchbadelektroden aus elektrisch leitfähigem Diamant ist bzw. hiermit beschichtet ist.

Die elektrochemische Reinigung kann auch noch durch weitere Maßnahmen beschleunigt bzw. verbessert werden. Eine Beschleunigung des Reinigungsvorganges tritt auf, wenn eine im Tauchbecken vorgesehene Heizvorrichtung die Flüssigkeit im Tauchbecken 22 erwärmt (vorzugsweise bis zu 90°C). Zusätzlich kann das Tauchbecken auch eine Vorrichtung zur Ultraschallerzeugung zur Beaufschlagung der Flüssigkeit mit Ultraschall aufweisen und so eine Beschleunigung des Reinigungsvogangs erreicht werden. Eine Beschleunigung kann auch durch die Beaufschlagung der Reinigungsflüssigkeit mit Luft-/Gasblasen oder durch Rühren erreicht werden.

Das oben beschriebene Verfahren ist auch zur (Vor)sterilisation geeignet. Für die verschmutzten Bereiche, welche nicht aus dem dotierten Diamantmaterial sind, ist noch eine Zusatzreinigung nötig.

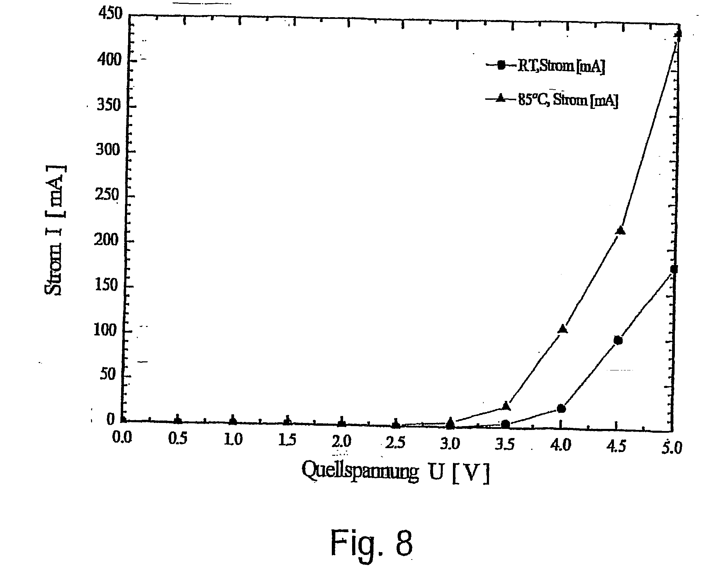

Schließlich zeigt Fig. 8 eine Elektrolyse-Strom-Spannungskurve für eine erfindungsgemäße Pinzette. Diese weist Kontaktbereiche auf, welche aus einem mit Bor dotierten Diamantmaterial bestehen, welches auf einen Kern aus Niob aufgebracht ist. Trotz relativ geringer Stromdichte ergab sich ein guter Reinigungseffekt.

## Patentansprüche

1. Instrument für chirurgische Zwecke, insbesondere zur Koagulation, welches mindestens einen Schenkel (2, 31) aufweist, wobei der mindestens eine Schenkel mit einem Steuergerät (12) elektrisch verbindbare elektrische Kontaktbereiche zur Einwirkung auf in der Nähe befindliches Gewebe (13) durch elektrische Beaufschlagung des Kontaktierbereiches aufweist,
**dadurch gekennzeichnet, dass** der Kontaktbereich aus zur Herstellung der elektrischen Leitfähigkeit dotiertem Diamant besteht und die Dotierstoffkonzentration des Kontaktbereiches (6) höher als 5 x 10¹⁸ cm⁻³ ist .

2. Instrument nach Anspruch 1, **dadurch gekennzeichnet, dass** der Bandabstand des Kontaktbereichs zwischen 5,2 eV und 5,6 eV, vorzugsweise zwischen 5,3 eV und 5,5 eV, liegt, besonders vorzugsweise 5,45 eV beträgt.

3. Instrument nach Anspruch 1 oder 2, **dadurch gekennzeichnet, dass** es zwei zueinander bewegbare Schenkel (2, 3) aufweist, z.B. in Form einer Pinzette (1), wobei die Spitze (4, 5) mindestens eines Schenkels den elektrischen Kontaktbereich aus dotiertem Diamant zum Berühren und Erfassen des Gewebes (13) zum Zwecke der Koagulation aufweist.

4. Instrument nach einem der vorhergehenden Ansprüche, **dadurch gekennzeichnet, dass** zumindest eine Schenkelspitze (4, 5) einen Kern (10) aufweist, auf welchen der die Kontaktbereiche bildende dotierte Diamant aufgebracht ist.

5. Instrument nach Anspruch 4, **dadurch gekennzeichnet, dass** das Material des Kerns elektrisch leitfähig ist, insbesondere dass der Kern aus einem refraktären Metall ist, bevorzugt Titan, Wolfram, Niob, Tantal, Iridium oder Legierungen dieser Metalle, oder dass der Kern Zr, Graphit oder kohlefaserverstärkter Kohlenstoff ist.

6. Instrument nach einem der Ansprüche 4 oder 5, **dadurch gekennzeichnet, dass** der Kontaktbereich aus dotiertem Diamant mittels Diamant-CVD auf dem Kern aufgebracht ist.

7. Instrument nach Anspruch 6, **dadurch gekennzeichnet, dass** der Kontaktbereich aus dotiertem Diamant mittels Hot-Filament-CVD, Mikrowellen-CVD oder electron-cyclotron-resonance-unterstützter Mikrowellen-CVD aufgebracht ist.

8. Instrument nach einem der vorhergehenden Ansprüche, **dadurch gekennzeichnet, dass** die Dotierstoffkonzentration des Kontaktbereiches (6) 5 x 10²⁰ cm⁻³ beträgt.

9. Instrument nach einem der vorhergehenden Ansprüche, **dadurch gekennzeichnet, dass** der spezifische Widerstand des dotierten Diamant der Kontaktbereiche (6) kleiner als 100 Ohm cm, bevorzugt kleiner als 1 Ohm cm, besonders bevorzugt kleiner als 0,01 Ohm cm ist.

10. Instrument nach einem der vorhergehenden Ansprüche, **dadurch gekennzeichnet, dass** der Dotierstoff des dotierten Diamant der Kontaktbereiche Bor, Schwefel, Stickstoff, Lithium, Phosphor oder Titan ist.

11. Instrument nach einem der vorhergehenden Ansprüche, **dadurch gekennzeichnet, dass** die Schichtdicke des Kontaktbereiches (6) weniger als 300 Mikrometer, bevorzugt weniger als 10 Mikrometer, besonders bevorzugt 1 bis 5 Mikrometer beträgt.

12. Instrument nach einem der Ansprüche 4 bis 11, **dadurch gekennzeichnet, dass** zwischen Kern (10) und Kontaktbereich (6) eine chemische Bindung besteht.

13. Instrument nach einem der Ansprüche 4 bis 12, **dadurch gekennzeichnet, dass** auf den Kern bereichsweise ein Isoliermaterial (11) aufgebracht ist.

14. Instrument nach Anspruch 13, **dadurch gekennzeichnet, dass** das Isoliermaterial auf dotiertem Diamant aufgebracht ist, wobei die Kontaktbereiche ausgelassen sind.

15. Instrument nach Anspruch 13, **dadurch gekennzeichnet, dass** das Isoliermaterial den dotierten Diamant vollständig bedeckt.

16. Instrument nach einem der Ansprüche 13 oder 15, **dadurch gekennzeichnet, dass** das Isoliermaterial (11) aus einer im CVD-Verfahren aufgebrachten nominell undotierten Diamantschicht aus texturiertem Diamant, nanokristallinem Diamant oder aus Diamond like Carbon besteht.

17. Instrument nach einem der vorhergehenden Ansprüche, **dadurch gekennzeichnet, dass** der dotierte Diamant aus in einem CVD-Verfahren aufgebrachtem texturiertem Diamant, nanokristallinem Diamant oder Diamond like carbon besteht.

18. Kombination aus einem Instrument nach einem der vorhergehenden Ansprüche mit einem Steuergerät, **dadurch gekennzeichnet, dass** das Steuergerät (12) mit einer Gleich- oder Wechselspannungsquelle, bevorzugt mit einem Hochfrequenz-Generator (14) zur Erzeugung unmodulierter und modulierter Hochfrequenzströme durch die Kontaktbereiche hindurch ausgestattet ist.

19. Kombination aus einem Instrument nach einem der vorhergehenden Ansprüche mit einem, mit Flüssigkeit füllbaren Tauchbecken und einer Spannungsquelle, **dadurch gekennzeichnet, dass** das Instrument mit dem Tauchbecken (22) zum Eintauchen der Kontaktbereiche (6, 7) in die Flüssigkeit und der Spannungsquelle (15) zum Anlegen einer Gleich- oder Wechselspannung an die in die Flüssigkeit eingetauchten Kontaktbereiche verbindbar ist.

20. Kombination nach Anspruch 19, **dadurch gekennzeichnet, dass** das Tauchbecken eine Heizvorrichtung zur Erwärmung der Flüssigkeit aufweist.

21. Kombination nach einem der Ansprüche 19 bis 20, **dadurch gekennzeichnet, dass** das Tauchbecken (22) einen Ultraschallerzeuger zur Beaufschlagung der Flüssigkeit mit Ultraschall aufweist.

22. Kombination nach einem der Ansprüche 19 bis 21, **dadurch gekennzeichnet, dass** das Tauchbecken eine destilliertes Wasser enthaltende Flüssigkeit zur Reinigung enthält.

23. Kombination nach Anspruch 22, **dadurch gekennzeichnet, dass** die Flüssigkeit destilliertes Wasser mit Zusätzen zur Erhöhung der elektrischen Leitfähigkeit wie Schwefelsäure ist.

24. Kombination nach Anspruch 22, **dadurch gekennzeichnet, dass** die Flüssigkeit destilliertes Wasser mit Reinigungsmitteln aufweist.

25. Instrument nach einem der vorhergehenden Ansprüche 1 bis 17, **dadurch gekennzeichnet, dass** zwei Kontaktbereiche gegeben sind und die beiden Kontaktbereiche (6, 7) eine entgegengesetzte oder eine gleichgerichtete Polung aufweisen.

26. Verfahren zur Reinigung der Kontaktbereiche des Instruments nach einem der vorhergehenden Ansprüche 1 bis 17 und 25, **dadurch gekennzeichnet, dass** diese zur elektrochemischen Reinigung in elektrisch leitende Flüssigkeit getaucht und mit einer Gleich- oder Wechselspannung zur Erzielung eines Stromflusses durch die Kontaktbereiche beaufschlagt werden, wobei die erzeugten Spannungen 1 bis 5 V betragen.

27. Verfahren zur Reinigung eines Instruments nach einem der vorhergehenden Ansprüche 1 bis 17 und 25, **dadurch gekennzeichnet, dass** die Polung der Kontaktbereiche im Reinigungsbetrieb unterschiedlich ist zu der Polung im Koagulationsbetrieb.

## Claims

1. Instrument for surgical applications, in particular for coagulation, comprising at least one shank (2, 31), and the at least one shank comprises electrical contact areas, which are electrically connectible to a control device (12) and which affect fabric (13) nearby by electrically impacting the contact area, **characterised in that** the contact area is composed of doped diamond for establishing electrical ductability, and the doped material concentration of the contact area (6) is higher than 5 x 10¹⁸ cm⁻³.

2. Instrument according to Claim 1, **characterised in that** the band gap of the contact area lies between 5.2 eV and 5.6 eV, preferably between 5.3 eV and 5.5 eV, particularly preferred 5.45 eV.

3. Instrument according to Claim 1 or 2, **characterised in that** it comprises two shanks (2, 3) which are movable towards each other, for example in the shape of pincers (1), and the tip (4, 5) of at least one shank comprises the electrical contact area of doped diamond for touching and holding the fabric (13) for the purpose of coagulation.

4. Instrument according to one of the above claims, **characterised in that** at least one shank tip (4, 5) comprises a core (10) on which is placed the doped diamond which establishes the contact areas.

5. Instrument according to Claim 4, **characterised in that** the material of the core is electrically ducting, in particular the core is made of a refractory metal, preferably titanium, tungsten, niobium, tantalum, iridium or alloys of these metals, or the core is Zr, graphite or carbonfibre reinforced carbon.

6. Instrument according to one of Claims 4 or 5, **characterised in that** the contact area of doped diamond is applied on the core by means of diamond-CVD.

7. Instrument according to Claim 6, **characterised in that** the contact area of doped diamond is applied by means of hot-filament-CVD, microwave-CVD or electron-cyclotron-resonance supported microwave-CVD.

8. Instrument according to one of the above claims, **characterised in that** the doping material concentration of the contact area (6) is 5 x 10²⁰ cm⁻³.

9. Instrument according to one of the above claims, **characterised in that** the specific resistance of doped diamond of the contact areas (6) is less than 100 ohm cm, preferably less than 1 ohm cm, particularly preferred less than 0.01 ohm cm.

10. Instrument according to one of the above claims, **characterised in that** the doping material of the doped diamond of the contact areas is boron, sulphur, nitrogen, lithium, phosphor or titanium.

11. Instrument according to one of the above claims, **characterised in that** the layer thickness of the contact area (6) is less than 300 micrometre, preferably less than 10 micrometre, particularly preferred between 1 and 5 micrometres.

12. Instrument according to one of Claims 4 to 11, **characterised in that** a chemical bond exists between the core (1) and the contact area (6).

13. Instrument according to one of Claims 4 to 12, **characterised in that** an insulating material (11) is applied to some core areas.

14. Instrument according to Claim 13, **characterised in that** the insulating material is applied to doped diamond whilst excluding the contact area.

15. Instrument according to Claim 13, **characterised in that** the insulating material fully covers the doped diamond.

16. Instrument according to one of Claims 13 or 15, **characterised in that** the insulating material (11) is composed of a nominally undoped diamond layer of textured diamond, nano-crystalline diamond or diamond like carbon, applied in a CVD process.

17. Instrument according to one of the above claims, **characterised in that** the doped diamond is composed of a textured diamond, nano-crystalline diamond or diamond like carbon, applied in a CVD process.

18. Combination of an instrument according to one of the above claims with a control device, **characterised in that** the control device (12) is equipped with a direct or alternating voltage source, preferably with a high-frequency generator (14) for generating unmodulated and modulated high-frequency currents through the contact areas.

19. Combination of an instrument according to one of the above claims with a liquid holding immersion tank and a voltage source, **characterised in that** the instrument with the immersion tank (22) for immersion of the contact areas (6, 7) into the liquid and the voltage source (15) for applying a direct or alternating current can be connected to the contact areas immersed in the fluid.

20. Combination according to Claim 19, **characterised in that** the immersion tank comprises a heating device for heating the liquid.

21. Combination according to one of Claims 19 to 20, **characterised in that** the immersion tank (22) comprises an ultrasound generator for loading the liquid with ultrasound.

22. Combination according to one of Claims 19 to 21, **characterised in that** the immersion tank contains a fluid including distilled water for cleaning purposes.

23. Combination according to Claim 22, **characterised in that** the liquid is distilled water with additives for increasing the electrical conductivity, such as sulphuric acid.

24. Instrument according to Claim 22, **characterised in that** the liquid includes distilled water with purification media.

25. Instrument according to one of the above claims 1 to 17, **characterised in that** two contact areas are given, and both contact areas (6, 7) are of opposite or equal polarisation.

26. Method for cleaning the contact areas of the instrument according to one of the above claims 1 to 17 and 25, **characterised in that** they are, for the purpose of electrochemical cleaning, dipped into an electrically conducting liquid and, for the purpose of establishing a current flow through the contact areas, charged with a direct or alternating voltage, and the generated voltages lie between 1 and 5 v.

27. Method for cleaning an instrument according to one of the above Claims 1 to 17 and 25, **characterised in that** the polarisation of the contact areas during cleaning operation differs from polarisation in coagulation operation.

## Revendications

1. Instrument destiné à des applications chirurgicales, en particulier pour la coagulation, qui présente au moins une branche (2, 3), la au moins une branche comportant des zones de contact électriques, pouvant être connectées par voie électrique à un appareil de commande (12), afin d'agir sur le tissu (13) se trouvant à proximité, en sollicitant par voie électrique la zone de contact,
**caractérisé en ce que** la zone de contact est constituée d'un diamant dopé permettant de produire la conductivité électrique, et la concentration en matière de dopage de la zone de contact (6) est supérieure à 5 x 10¹⁸ cm⁻³.

2. Instrument selon la revendication 1, **caractérisé en ce que** l'intervalle d'énergie entre deux bandes de la zone de contact est compris entre 5,2 eV et 5,6 eV, de préférence entre 5,3 eV et 5,5 eV, et de manière particulièrement préférée, est égale à 5,45 eV.

3. Instrument selon l'une quelconque des revendications 1 ou 2, **caractérisé en ce qu'**il présente deux branches (2, 3) mobiles l'une par rapport à l'autre, se présentant par exemple sous la forme d'une pince (1), la pointe (4, 5) d'au moins une branche présentant la zone de contact électrique constituée de diamant dopé, permettant d'établir le contact avec le tissu (13) et de le saisir, à des fins de coagulation.

4. Instrument selon l'une quelconque des revendications précédentes, **caractérisé en ce qu'**au moins une pointe de branche (4, 5) présente une âme (10) sur laquelle est appliqué le diamant dopé, formant les zones de contact.

5. Instrument selon la revendication 4, **caractérisé en ce que** la matière de l'âme est électroconductrice, en particulier **en ce que** l'âme est réalisée dans un métal réfractaire, de préférence du titane, du tungstène, de la niobite, du tantale, de l'iridium ou des alliages de ces métaux, ou **en ce que** l'âme est en Zr, graphite ou autre carbone renforcé par des fibres.

6. Instrument selon l'une quelconque des revendications 4 ou 5, **caractérisé en ce que** la zone de contact constituée de diamant dopé est appliquée sur l'âme à l'aide d'un dépôt chimique en phase vapeur de diamant.

7. Instrument selon la revendication 6, **caractérisé en ce que** la zone de contact constituée de diamant dopé est appliquée à l'aide d'un dépôt en phase vapeur par fil chaud, dépôt en phase vapeur par micro-ondes ou dépôt en phase vapeur par micro-ondes à résonance cyclotron électronique.

8. Instrument selon l'une quelconque des revendications précédentes, **caractérisé en ce que** la concentration en matière dopée de la zone de contact (6) est égale à 5 x 10²⁰ cm⁻³.

9. Instrument selon l'une quelconque des revendications précédentes, **caractérisé en ce que** la résistance spécifique du diamant dopé des zones de contact (6) est inférieure à 100 ohms / cm, de préférence inférieure à 1 ohm / cm, et de manière particulièrement préférée inférieure à 0,01 ohm / cm.

10. Instrument selon l'une quelconque des revendications précédentes, **caractérisé en ce que** la substance dopée du diamant dopé des zones de contact est du bore, du soufre, de l'azote, du lithium, du phosphore ou du titane.

11. Instrument selon l'une quelconque des revendications précédentes, **caractérisé en ce que** l'épaisseur de couche de la zone de contact (6) est inférieure à 300 micromètres, de préférence inférieure à 10 micromètres, et de manière particulièrement préférée, comprise entre 1 et 5 micromètres.

12. Instrument selon l'une quelconque des revendications 4 à 11, **caractérisé en ce que**, entre l'âme (10) et la zone de contact (6), il existe une liaison chimique.

13. Instrument selon l'une quelconque des revendications 4 à 12, **caractérisé en ce que**, sur l'âme, on applique, zone par zone, une matière isolante (11).

14. Instrument selon la revendication 13, **caractérisé en ce que** la matière isolante est appliquée sur le diamant dopé, moyennant quoi les zones de contact sont omises.

15. Instrument selon la revendication 13, **caractérisé en ce que** la matière isolante recouvre totalement le diamant dopé.

16. Instrument selon l'une quelconque des revendications 13 ou 15, **caractérisé en ce que** la matière isolante (11) est constituée d'une couche de diamant non dopée normalement appliquée grâce à un procédé de dépôt chimique en phase vapeur de diamant texturé, diamant nanocristallin ou carbone sous forme de diamant amorphe.

17. Instrument selon l'une quelconque des revendications précédentes, **caractérisé en ce que** le diamant dopé est constitué d'un diamant texturé, diamant nanocristallin ou carbone sous forme de diamant amorphe, appliqué grâce à un procédé de dépôt chimique en phase vapeur.

18. Combinaison entre un instrument selon l'une quelconque des revendications précédentes et un appareil de commande, **caractérisé en ce que** l'appareil de commande (12) est doté d'une source de tension continue ou alternative, de préférence d'un générateur de hautes fréquences (14) permettant de produire des courants haute fréquence non modulés et modulés, au travers des zones de contact.

19. Combinaison d'un instrument selon l'une quelconque des revendications précédentes avec un récipient d'immersion pouvant être rempli de liquide et une source de tension, **caractérisée en ce que** l'instrument peut être raccordé aux récipients d'immersion (22) pour plonger les zones de contact (6, 7) dans le liquide et à la source de tension (15) pour établir une tension continue ou alternative au niveau des zones de contact plongées dans le liquide.

20. Combinaison selon la revendication 19, **caractérisée en ce que** le récipient d'immersion présente un dispositif de chauffage permettant de chauffer le liquide.

21. Combinaison selon l'une quelconque des revendications 19 et 20, **caractérisée en ce que** le récipient d'immersion (22) présente un générateur d'ultrasons, permettant de solliciter le liquide avec des ultrasons.

22. Combinaison selon l'une quelconque des revendications 19 à 21, **caractérisée en ce que** le récipient d'immersion contient un liquide avec de l'eau distillée à des fins de nettoyage.

23. Combinaison selon la revendication 22, **caractérisée en ce que** le liquide est de l'eau distillée avec des additifs pour faire augmenter la conductivité électrique, tel que l'acide sulfurique.

24. Combinaison selon la revendication 22, **caractérisée en ce que** le liquide contient de l'eau distillée avec des agents nettoyants.

25. Instrument selon l'une quelconque des revendications 1 à 17, **caractérisé en ce qu'**il existe deux zones de contact, et les deux zones de contact (6, 7) présentent une polarisation dans un sens identique ou inverse.

26. Procédé de nettoyage des zones de contact de l'instrument selon l'une quelconque des revendications 1 à 17 et 25, **caractérisée en ce que** ces zones de contact sont plongées dans le liquide électroconducteur à des fins de nettoyage électrochimique, et sont sollicités avec une tension continue ou alternative afin d'obtenir une circulation du courant au travers des zones de contact, les tensions produites allant de 1 à 5 V.

27. Procédé de nettoyage d'un instrument selon l'une quelconque des revendications 1 à 17 et 25, **caractérisé en ce que** la polarisation des zones de contact lors de l'opération de nettoyage est différente de la polarisation lors de l'opération de coagulation.
